# EUROPÄISCHE PATENTSCHRIFT

(11) **EP 1 343 011 B1**
(45) Veröffentlichungstag und Bekanntmachung des Hinweises auf die Patenterteilung: **22.04.2009**
(21) Anmeldenummer: 03003954.9
(22) Anmeldetag: 21.02.2003
(51) Int. Cl.: G01N 33/543, C12M 1/34, G01N 27/403, G01N 27/416, C12Q 1/68, B29D 7/01

(54) **Vorrichtung zum elektrochemischen Nachweis einer Nukleotidsequenz, Analyse-Kassette für eine solche Vorrichtung und Verfahren zur Herstellung einer solchen Analyse-Kassette**
Device for electrochemical detection of nucleotide sequences, analysis-cassette module specially adapted to the device and process of manufacture thereof
Dispositif de détection des séquences de nucléotides, cassette d'analyse accessoire et procédé de fabrication de la cassette d'analyse

(30) Priorität: 07.03.2002 DE 10210051
(43) Veröffentlichungstag der Anmeldung: 10.09.2003
(73) Patentinhaber: SIEMENS AKTIENGESELLSCHAFT, 80333 München (DE)
(72) Erfinder: Knoche, Jochem, 91056 Erlangen (DE)

(56) Entgegenhaltungen:
- WO-A-01/16361
- US-A- 5 972 692
- US-A- 6 063 259
- PALECEK E: "FROM POLAROGRAPHY OF DNA TO MICROANALYSIS WITH NUCLEIC ACID-MODIFIED ELECTRODES" ELECTROANALYSIS, VHC PUBLISHERS, INC, US, Bd. 8, Nr. 1, 1996, Seiten 7-14, XP000973887 ISSN: 1040-0397

## Beschreibung

Vorrichtung zum elektrochemischen Nachweis einer Nukleotidsequenz, Analyse-Kassette für eine solche Vorrichtung und Verfahren zur Herstellung einer solchen Analyse-Kassette

Die Erfindung bezieht sich auf eine Vorrichtung zum elektrochemischen Nachweis einer in einer Flüssigkeit befindlichen Nukleotidsequenz, mit einer Arbeitselektrode, an die ein Biopolymer gebunden ist, welches eine spezifische Affinität zu der nachzuweisenden Nukleotidsequenz aufweist, und mit einer Gegenelektrode.

Die Erfindung betrifft außerdem eine Analyse-Kassette für den elektrochemischen Nachweis einer in einer Flüssigkeit befindlichen Nukleotidsequenz und zur Verwendung mit einer solchen Vorrichtung sowie ein Zusatzmodul für einen tragbaren Computer.

Außerdem liegt im Rahmen der Erfindung noch ein Herstellungsverfahren für eine solche Analyse-Kassette.

Aus der DE 41 39 121 C1 ist eine Vorrichtung zum Messen der Ionen-Konzentration bekannt, die einen Messeinsatz aufweist, der auswechselbar in ein Basisgerät einsetzbar ist. Die zu untersuchende Flüssigkeit wird hierzu in den Messeinsatz gegeben. In WO 98/05958 ist eine Vorrichtung zur austauschbaren Aufnahme von Messkartuschen oder Messzellen zur Bestimmung biochemischer Messparamter beschrieben. Die Vorrichtung ist mit einem computergesteuerten Analysesystem verbunden.

Im Bereich der Medizintechnik können diverse Erkrankungen oder Fehlentwicklungen nachgewiesen werden, indem durch die Erkrankung oder die Fehlentwicklung in einer Körperflüssigkeit oder im Gewebe verursachte genetisch signifikante Spuren nachgewiesen werden. Beispielsweise hinterlassen Krankheitserregende Bakterien oder Viren Bruchstücke ihrer DNA, d.h.

Nukleotide oder Sequenzen hiervon, das sind Nukleotidsequenzen, Oligo-Nukleotidsequenzen oder Polynukleotidsequenzen, im Blut oder Urin. Bei verschiedenen Arten von Krebserkrankungen lassen sich Teile der erkrankten Krebszellen mit ihren genetisch signifikanten Defekten in Körperflüssigkeiten nachweisen. Eine ähnliche Aufgabenstellung besteht im Bereich der Lebensmittelchemie, wo darauf abgezielt wird, die für das Verderben von Lebensmitteln verantwortlichen Bakterien durch Nachweis ihrer genetisch signifikanten Spuren zu identifizieren. Die Aufgabe des Nachweises genetischer Spuren stellt sich auch bei der Untersuchung von Lebensmitteln im Hinblick auf deren Herkunft, insbesondere um Aufklärung darüber zu erhalten, ob in den Lebensmitteln vorhandene Bestandteile aus natürlichen Pflanzen oder aus genmanipulierten Pflanzen stammen.

Die Erfindung zielt darauf ab, eine Vorrichtung zum elektrochemischen Nachweis von beispielsweise aus solchen Aufgabenstellungen stammenden Nukleotidsequenzen anzugeben.

Gemäß einer bekannten Vorgehensweise wird dem Patienten eine Blutprobe entnommen, der ein Gerinnungshemmer zugesetzt wird, sowie eine Substanz, welche die Zellwände aufbricht. Dazu kommt ein spezielles Enzym, das die nach Aufbrechen der Zellwände freigesetzten DNA-Stränge an genau vordefinierten Stellen auftrennt ("enzymatische Schere").

Nach diesem ersten Schritt wird in der Regel in einem zweiten Schritt eine Polymerase-Ketten-Reaktion (PCR) durchgeführt, um die Empfindlichkeit bei dem nachfolgenden Nachweisschritt zu erhöhen. Ein solches Verfahren ist beschrieben in DE 198 26 153 A1. Bei der Polymerase-Ketten-Reaktion wird eine Replikation oder Vervielfachung der in der Lösung oder Flüssigkeit vorhandenen DNA-Abschnitte angeregt. Die hierzu erforderlichen molekularen Bausteine, das sind die vier am Aufbau der DNA beteiligten Aminosäuren, werden der Probe zugefügt. Die Probe wird dann kontrolliert erwärmt und anschließend wieder abgekühlt. Gemäß der bereits zitierten DE 198 26 153 A1 wird der Nachweisprozess, d.h. die dritte Stufe, durch ein Fluoreszenz-basiertes Verfahren durchgeführt.

Zum Nachweis der gesuchten DNA-Abschnitte sind auch elektrochemische Verfahren bekannt, beispielsweise aus der deutschen Offenlegungsschritt 199 40 647 A1, der US-Patentschrift US 5,312,527 und der US-Patentschrift US 5,871,918.

Elektrochemische Nachweisverfahren sind zudem beschrieben in der WO 01/42508 A2 und WO 95/12808. Insbesondere das in der letztgenannten Schrift offenbarte System ist sehr aufwendig und nur bei Einsatz in Großlabors wirtschaftlich- verwendbar.

Bei den elektrochemischen Nachweisverfahren werden die gesuchten DNA-Abschnitte oder Nukleotidsequenzen in der Lösung mit speziellen Arbeitselektroden nachgewiesen, die mit speziellen Rezeptor-Molekülen oder Biopolymeren benetzt sind, d.h. mit passenden "Gegenstücken" zu den gesuchten DNA-Abschnitten oder Nukleotidsequenzen. An der in die Flüssigkeit eingetauchten Arbeitselektrode lagern sich nur die exakt passenden Nukleotidsequenzen an, so dass anschließend mittels einer elektrischen Auswertung die Anlagerung nachweisbar ist.

Ein hierzu verwendetes Verfahren ist die PSA ("potentiometric stripping analysis"). Dabei wird zwischen der Arbeitselektrode und einer Gegenelektrode ein Strom eingeprägt. Zwischen der Arbeitselektrode und einer Referenzelektrode kann dann eine allmählich ansteigende Spannung gemessen werden. Haben sich an der Arbeitselektrode Nukleotidsequenzen der gesuchten Art angelagert, ist ein Verharren des Spannungsanstiegs bei einem bestimmten Wert, z.B. bei 0,8 V, zu beobachten, da bei dieser Spannung beispielsweise das im gesuchten DNA-Abschnitt enthaltene Guanin oxidiert. Die zeitliche Breite dieses Spannungsplateaus kann als Maß für die oxidierte Guanin-Menge verwendet werden, woraus der Rückschluß möglich ist, ob in der untersuchten Probe DNA-Abschnitte der gesuchten Art in diagnostisch relevanter Menge vorhanden waren.

Die bekannten, nach derartigen Prozessschritten arbeitenden Geräte sind Geräte der Laborchemie. Sie sind aufgrund ihrer Größe nur für den ortsfesten Einsatz geeignet. Die Geräte enthalten in der Regel aufwendige Komponenten zum Transport und zur Dosierung der Flüssigkeit und zum anschließenden Säubern der Analysekammer. Die Komponenten umfassen Vorratsbehälter, Ventile, Schläuche, Pumpen und dergleichen. Auch hierdurch ist ein hoher Platzbedarf begründet. Außerdem verlangen diese Komponenten einen beträchtlichen Wartungsaufwand, da die Anforderungen an die Sauberkeit der Fluide auf sehr hohem Niveau liegen. Da die derzeit üblichen Geräte nur im Labor einsetzbar sind, müssen entsprechende Proben häufig erst noch zu einem entfernten zentralen Labör transportiert werden. Dies ist insbesondere in der Medizin bei Erkrankungen mit akutem Verlauf von großem Nachteil.

US 6,063,259 offenbart einen miniaturisierten Dickfilm Sensor für die Bestimmung von Nukleinsäuren mittels potentiometric stripping analysis (PSA). Der Sensor besteht aus zwei oder drei Elektroden. Die Sensoreinheit kann in Verbindung mit einem handlichen, batteriebetriebenen Computer-Auswertungsgerät verwendet werden. Jedoch kann in diesem Gerät keine PCR Analyse vorgenommen werden.

Der Erfindung liegt die Aufgabe zugrunde, eine Vorrichtung zum elektrochemischen Nachweis einer Nukleotidsequenz anzugeben, die am Ort der Probennahme, z.B. vor Ort beim Patienten, dem sogenannten "point of care", einsetzbar ist und unmittelbar dort ein Nachweisergebnis ausgibt. Hierzu sollen auch Hilfsmittel und ein Herstellungsverfahren für ein Hilfsmittel angegeben werden, die eine kostengünstige Herstellung der Vorrichtung erlauben, um die Vorrichtung in großer Stückzahl für den dezentralen Einsatz zu fertigen.

Diese Aufgabe wird bezogen auf die eingangs genannte Vorrichtung gemäß der Erfindung gelöst durch
a) eine austauschbare Analyse-Kassette zum Einfüllen der Flüssigkeit, wobei die Arbeitselektrode und die Gegenelektrode in der Analyse-Kassette angeordnet und mit der Flüssigkeit in Kontakt bringbar sind,
b) eine tragbare, mit der Analyse-Kassette elektrisch verbindbare Computereinrichtung mit einem Display und mit einer netzunabhängigen Energiequelle, wobei die Computereinrichtung derart ausgebildet ist, dass den Elektroden eine Spannung und/oder Strom aufprägbar ist, dass ein von der Anlagerung der nachzuweisenden Nukleotidsequenz auf oder an oder in der Arbeitselektrode herrührendes Stromund/oder Spannungssignal erfassbar und dass ein daraus resultierendes Nachweisergebnis auf dem Display darstellbar ist, und
c) ein aus der Energiequelle gespeistes Temperierelement zum Kühlen und/oder Beheizen der Flüssigkeit in der Kassette.

Unter einer tragbaren Computereinrichtung wird im Zusammenhang mit der Erfindung jede Computereinrichtung verstanden, die für den mobilen Einsatz vorgesehen ist, z.B. auch ein Laptop, Notebook, Palmtop oder "Handheld".

Der Erfindung liegt unter anderem die Erkenntnis zugrunde, dass die eingangs geschilderten elektrochemischen Nachweisverfahren auch mittels einer tragbaren Vorrichtung durchführbar sind. Mit der Vorrichtung sind beispielsweise PCR und PSA wie eingangs beschrieben dezentral durchführbar.

Außerdem basiert die erfindungsgemäße Vorrichtung auf der Überlegung, aktive Komponenten zum Transport, Umwälzen oder Dosieren der Flüssigkeit zu vermeiden. Statt dessen ist eine austauschbare Analyse-Kassette vorhanden, die vorzugsweise als Einmal-Kassette, als Wegwerf- oder Verbrauchsmaterial ausgestaltet ist. Dadurch entfällt die Notwendigkeit einer regelmäßigen Reinigung und/oder Wartung entsprechender Komponenten. Durch die Analyse-Kassette einerseits und die Computereinrichtung andererseits ist die Vorrichtung nach der Erfindung in funktionell vorteilhafter Weise getrennt in einen auswechselbaren Nassteil ("Chemiemodul") und in einen längerfristig verwendbaren elektrischen Teil.

Mit der Vorrichtung nach der Erfindung ist es in vorteilhafter Weise möglich, auch für einen nicht speziell in Laborchemie geschulten Anwender vor Ort beim Patienten oder bei einer lebensmittelchemischen Probenentnahme Nukleotidsequenzen elektrochemisch nachzuweisen. Die Zeit für den Transport von Proben zu einem zentralen Labor entfällt. Dies bedeutet auch eine beträchtliche Kosteneinsparung.

Durch das Temperierelement ist es in besonders einfacher Weise möglich, die Probenflüssigkeit, die hierzu mit entsprechenden Aminosäuren versetzt wurde, einer Polymerase-Ketten-Reaktion (PCR) zu unterziehen, wie sie eingangs geschildert wurde. Die Einhaltung entsprechender Erwärmungs- und Abkühlphasen kann dabei von der Computereinrichtung gesteuert werden.

Vorzugsweise ist zum Aufprägen der Spannung bzw. des Stroms die Energiequelle der Computereinrichtung verwendet. Eine weitere Erkenntnis besteht nämlich darin, dass die bei einer tragbaren Computereinrichtung ohnehin vorhandene hetzunabhängige Energiequelle gleichzeitig der Durchführung des elektrochemischen Nachweisverfahrens dienen kann. Die netzunabhängige Energiequelle der tragbaren Computereinrichtung ist insbesondere eine elektrische Batterie oder ein elektrischer Akkumulator.

Das Temperierelement ist vorzugsweise ein Peltierelement.

Die Computereinrichtung weist vorzugsweise sowohl einen Computer mit einem Eingabemittel, insbesondere mit Taste(n), und dem Display sowie ein an eine digitale Schnittstelle des Computers anschließbares Zusatzmodul auf, das mit der Analyse-Kassette über eine analoge Schnittstelle verbindbar ist. Auf dem Zusatzmodul können beispielsweise Digital-Analogwandler und Analog-Digitalwandler angebracht sein. Durch die Aufteilung der Computereinrichtung in den Computer einerseits und das Modul andererseits ist es in vorteilhafter Weise möglich, für unterschiedliche Anwendungen Zusatzmodule mit unterschiedlichen elektrischen Anforderungen in ein und denselben vor Ort vorhandenen Computer einzubauen.

Die digitale Schnittstelle ist insbesondere eine PCMCIA-Schnittstelle ("Personal Computer Memory Card International Association"). Eine entsprechende Schnittstelle an dem Computer erlaubt beispielsweise das Einführen von so genannten PCMCIA-Steckkarten im Scheckkartenformat.

Mit besonderem Vorteil ist der Computer ein mobiler Standard-Computer, insbesondere ein Laptop, ein Notebook oder ein während der Bedienung zum Halten in der Hand vorgesehener Computer, z.B. ein Palmtop oder "Handheld". In dieser Ausführung wird die Aufteilung der Computereinrichtung sowohl in einen Computer als auch in ein Zusatzmodul als besonders vorteilhaft deutlich, da z.B. bei dem Patienten vor Ort von dem behandelnden Arzt ein ohnehin vorhandener Computer verwendbar ist, der auch andere Aufgaben übernehmen kann. Dadurch werden die Investitionskosten in die Vorrichtung nach der Erfindung gering gehalten und die Vorrichtung wird für einen breiten Einsatz besonders geeignet.

Das Zusatzmodul ist vorzugssweise derart ausgeführt, dass es zumindest teilweise in ein Gehäuse des Computers einsteckbar ist. Beispielsweise hat es endseitig das Format einer PCMCIA-Steckkarte.

Außerdem bevorzugt weist das Zusatzmodul eine Ausnehmung oder Öffnung zum Einschieben der Analyse-Kassette auf. Das Zusatzmodul kann beispielsweise als Andockstation für die Analyse-Kassette dienen.

Das erwähnte Temperier-Element ist vorzugsweise im Zusatzmodul angebracht. Dort ist es insbesondere derart eingebaut, dass es in großflächigen thermischen Kontakt mit der eingeschobenen oder angedockten Analyse-Kassette kommt.

Nach einer besonders bevorzugten Weiterbildung weist die Vorrichtung ein im Zusatzmodul angebrachtes, elektrisch betreibbares Mittel zum Ausüben einer Kraft auf die Analyse-Kassette auf. Diese Kraft kann in eine Bewegung der Arbeitselektrode und/oder der Gegenelektrode umgesetzt werden. Dem liegt die Überlegung zugrunde, dass es für die Prozesssicherheit der Analyse von Vorteil ist, wenn während der Aufheiz- und Abkühlphasen der bereits beschriebenen PCR die Arbeitselektrode noch nicht von der Flüssigkeit oder Lösung benetzt wird. Vielmehr soll die Arbeitselektrode erst nach Ablauf der PCR zur Durchführung der elektrochemischen Analyse in die Flüssigkeit oder Lösung eintauchen.

Besonders bevorzugt ist dabei, dass das Mittel zum Ausüben einer Kraft ein Element mit einer Formgedächtnislegierung umfasst, wobei das Element insbesondere bei einer Erwärmung die Rückkehr in einen Herstellungs-bedingten Ausgangszustand anstrebt und dabei die Kraft erzeugt. Formgedächtnislegierungen sind auch unter der Bezeichnung "shape memory alloy" bekannt (z.B. Nitinol, eingetragenes Warenzeichen). Es existieren Formgedächtnislegierungen, die bei Raumtemperatur leicht verformbar sind, bei Erwärmung jedoch in eine Form zurückkehren, die ihnen während ihrer Herstellung durch eine spezielle Behandlung vorgegeben wurde.

Zum Erwärmen des Formgedächtniselements ist es vorteilhaft, falls dieses als Draht oder Band ausgebildet ist, so dass infolge des sich daraus ergebenden elektrischen Widerstandes eine elektrische Spannung oder ein elektrischer Strom direkt an das Band bzw. den Draht anlegbar ist.

Das Mittel zum Ausüben einer Kraft ist mit Vorteil derart im Zusatzmodul angeordnet, dass das Mittel bei Einschieben der Austauschkassette in die Ausnehmung im Zusatzmodul gestaucht wird. Dies hat den Vorteil, dass ein gesonderter Rückstellmedanismus nicht erforderlich ist. Die Rückstellkraft wird vielmehr von der Person aufgebracht, welche die Analyse-Kassette in das Zusatzmodul einsteckt oder einführt.

Nach einer anderen bevorzugten Weiterbildung ist die Computereinrichtung derart ausgebildet, dass unter Verwendung des erfassten Strom- und/oder Spannungssignals eine Diagnose hinsichtlich eines in der Flüssigkeit befindlichen Krankheitserregers oder hinsichtlich einer in der Flüssigkeit befindlichen krankhaften Körperzelle erstellbar und auf dem Display anzeigbar ist. Die entsprechende Auswertung übernimmt eine in den Computer geladene Software, welche beispielsweise zum Nachweis oder zur Diagnose erforderliche Entscheiungsdaten einer lokalen Datenbank in der Computereinrichtung oder einer über ein Datennetz angebundenen entfernten Datenbank entnimmt. Die Anbindung an die entfernte Datenbank kann drahtlos über Funknetze, wie z.B. WaveLAN, GSM oder UMTS, geschehen.

Beschrieben wird auch eine Analyse-Kassette als solche, die insbesondere als Hilfs- oder Austauschmittel für die oben beschriebene Vorrichtung geeignet ist. Die Analyse-Kassette für den elektrochemischen Nachweis einer in einer Flüssigkeit befindlichen Nukleotidsequenz weist ein dichtes Gehäuse auf, in welchem eine Arbeitselektrode mit einem daran gebundenen, eine spezifische Affinität zu der nachzuweisenden Nukleotidsequenz aufweisenden Biopolymer und eine Gegenelektrode angeordnet sind, wobei das Gehäuse ein ausflussfreies Zufuhrmittel aufweist, um die Flüssigkeit zu den Elektroden im Gehäuse zu bringen. Die Analyse-Kassette kann in vorteilhafter Weise als Einmal-Kassette oder Austauschteil, welches nach einmaligem Gebrauch zu entsorgen ist, ausgestaltet sein.

Die Analyse-Kassette, die mit einer tragbaren Computereinrichtung elektrisch verbindbar ist, hat den Vorteil, dass der Nassteil eines elektrochemischen Nachweisverfahrens funktioneil vom elektronischen Auswerteteil, der in der Computereinrichtung angeordnet ist, trennbar ist. Dadurch ist es im mobilen Einsatz vor Ort möglich, mit der tragbaren Computereinrichtung unter Verwendung unterschiedlicher oder gleicher Einmal-Kassetten nacheinander mehrere unterschiedliche oder gleiche elektrochemische Nachweisverfahren für eine in einer Flüssigkeit befindliche Nukleotidsequenz durchzuführen.

Die Arbeitselektrode und die Gegenelektrode sind vorzugsweise derart ausgeführt, wie es oben im Zusammenhang mit der Vorrichtung bereits erläutert wurde. Für bevorzugte Ausgestaltungen, die die Anpassung an die tragbare Computereinrichtung betreffen, wird ebenfalls auf die oben bereits gemachten Ausführungen verwiesen.

Das Gehäuse der Analyse-Kassette weist vorzugsweise ein Oberteil mit daran befestigter Arbeitselektrode und ein-Unterteil mit der Gegenelektrode auf.

Zum simultanen Nachweis mehrerer voneinander unterschiedlicher Nukleotidsequenzen können mehrere Arbeitselektroden mit voneinander unterschiedlichen Biopolymeren vorhanden sein.

Jede Arbeitselektrode kann mit dem Biopolymer an der Oberfläche beschichtet und/oder mit dem Biopolymer getränkt sein.

Im Unterteil des Gehäuses der Analyse-Kassette sind vorzugsweise mehrere Vertiefungen mit jeweils einer darin angeordneten Gegenelektrode gebildet. Damit kann das zur Analyse notwendige Flüssigkeitsvolumen gering gehalten werden.

Nach einer besonders bevorzugten Weiterbildung weist das Gehäuse einen stauchbaren Bereich auf, wobei bei Ausüben einer Kraft auf die Analyse-Kassette die Arbeitselektrode unter Stauchung des Bereichs in Richtung zum Unterteil bewegbar ist. Eine solche Kraft kann von außen beispielsweise vom Nutzer aufgebracht werden. In bevorzugter Ausführung wird diese Kraft jedoch von dem oben bereits erläuterten, im Zusatzmodul angebrachten elektrisch betreibbaren Mittel ausgeübt. Durch die Stauchbewegung ist es möglich, die Arbeitselektrode während der für die Polymerase-Ketten-Reaktion notwendigen Aufheiz- und Abkühlphasen noch unbenetzt von der Flüssigkeit zu belassen. Erst nach Beendigung der Temperierphasen wird ein oberer Bereich des Gehäuses durch Ausüben der Kraft in Richtung Unterteil bewegt, wodurch die Arbeitselektrode mit der in das Gehäuse injizierten Flüssigkeit in Kontakt kommt. Dadurch wird die Prozesssicherheit der Analyse in vorteilhafter Weise erhöht.

Als stauchbäres Verbindungsmittel ist vorzugsweise ein Faltenbalg vorhanden.

Nach einer anderen vorzugsweisen Weiterbildung weist die Analyse-Kassette ein von der Computereinrichtung auslesbares Speichermodul auf, in dem die Art der Arbeitselektrode und optional Angaben über die Haltbarkeit,den Hersteller und/oder die Herstellungsserie eingeprägt sind. Dadurch ist vermeidbar, dass Analyse-Kassetten mit Arbeitselektroden für unterschiedliche Nachweiszwecke unbeabsichtigt vertauscht werden. Hierzu ist das Speichermodul außerdem vorzugsweise nicht beschreibbar, sondern nur auslesbar. Die auf dem Speichermodul abgelegten Daten können beispielsweise über eine entsprechende Kontaktierung von der Computereinrichtung ausgelesen und am Display angezeigt werden.

In dem Speichermodul sind vorzugsweise auch Daten enthalten, welche Prozessparameter, insbesondere arbeitselektrodenspezifische Prozessparameter, definieren. Beispielsweise sind für alle in der spezifischen Analyse-Kassette vorhandenen Arbeitselektroden die Schwellwerte für eine Positiv-/Negativ-Aussage des Nachweises angegeben. Auch PCR-Parameter wie z.B. die Anzahl der zu durchlaufenden PCR-Zyklen sind dort ablegbar. DNA-Sequenzen des Human-Genoms sind nämlich in der Regel in derartiger Konzentration in den Körperflüssigkeiten vorhanden, dass eine PCR schlicht überflüssig ist, wogegen die meisten Viren mehrere PCR-Durchläufe und somit eine 2ⁿ-fache Vervielfältigung brauchen, um nachweisbar zu werden. Mit dieser Information im Speichermodul ist sichergestellt, dass die Vorrichtung auch Analyse-Kasetten auswerten kann, die zum Zeitpunkt der Geräteherstellung noch nicht definiert waren. Es entfallen also aufwendige Nachrüstaktionen vor Ort beim Kunden und es kann auf aktuelle Situationen, wie dem Ausbruch neuer Krankheiten, extrem schnell reagiert werden.

Das ausflussfreie Zufuhrmittel umfasst insbesondere ein elastisches Wandelement, welches mit einer Kanüle durchstechbar ist.

Im Rahmen der Beschreibung liegt auch noch die Erkenntnis, einen Satz mehrerer baugleicher Analyse-Kassetten der genannten Art vorzusehen, wobei jede Analyse-Kassette eine oder mehrere Arbeitselektrode(n) zum Nachweis einer bzw. mehrerer Nukleotidsequenzen aufweist und wobei sich die Analyse-Kassetten dadurch voneinander unterscheiden, dass sie zum Nachweis voneinander unterschiedlicher Nukleotidsequenzen bzw. zum Nachweis voneinander unterschiedlicher Kombinationen von Nukleotidsequenzen geeignet sind. Mit einem solchen Satz von Analyse-Kassetten ist es dem medizinischen Personal vor Ort beim Patienten oder einem Lebensmittelchemiker in einfacher Weise möglich, ein breites Anwendungsspektrum abzudecken. Mit anderen Worten: Es genügt, wenn der Arzt oder Chemiker nur eine tragbare Computereinrichtung bei sich hat sowie einen Satz in Frage kommender, beispielsweise nur handgroßer, Analyse-Kassetten. Die Analyse-Kassette kann - beispielsweise in gemeinsamer Verpackung - zusammen mit Ampullen mit Lösungen zur Probenvorbereitung, mit einer Einmal-Spritze, mit einem Einmal-Handschuh und ähnlichen Hilfsmitteln oder einer Teilmenge hiervon als "kit-of-parts" zusammengestellt oder lieferbar sein.

Vorzugsweise sind die unterschiedlichen Analyse-Kassetten für unterschiedliche Diagnosen hinsichtlich eines in der Flüssigkeit befindlichen Krankheitserregers oder hinsichtlich einer in der Flüssigkeit befindlichen krankhaften Körperzelle präpariert. Beispielsweise ist eine Analyse-Kassette für einen Grippe-Nachweis hergerichtet und trägt eine entsprechende Aufschrift. Eine solche Kassette weist z.B. Arbeitselektroden zum Nachweis unterschiedlicher Virus-Stämme auf.

Nach einer besonders bevorzugten Weiterbildung des Satzes sind dessen unterschiedliche Analyse-Kassetten hinsichtlich ihres potentiellen Diagnoseergebnisses für eine hierarchische Vorgehensweise präpariert.

Beispielsweise ist wenigstens eine erste Analyse-Kassette für eine Grobdiagnose vorhanden, der eine oder mehrere weitere Analyse-Kassetten für eine Feindiagnose nachgeordnet sind, wobei optional die weiteren Analyse-Kassetten in Abhängigkeit vom Ergebnis der Grobdiagnose auszuwählen sind. Beispielsweise wird mit der ersten Analyse-Kassette eine Grobklassifikation nach Viren oder Bakterien als Krankheitsverursachern vorgenommen, und dann bei Bedarf mit weiteren Analyse-Kassetten anderen Typs die genaue Art des Krankheitserregers ermittelt.

Offenbart wird hier auch ein Zusatzmodul zum Anschluss an eine digitale Schnittstelle eines tragbaren Computers, mit einer analogen Schnittstelle zum Anschließen einer austauschbaren Analyse-Kassette, mit einer elektronischen Schaltungsanordnung, die dazu hergerichtet ist, in der Analyse-Kassette befindlichen Elektroden eine Spannung und/oder einen Strom aufzuprägen und ein infolge einer elektrochemischen Reaktion in der Analyse-Kassette entstehendes Strom- und/oder Spannungssignal zu konditionieren, und mit einem Temperierelement zum Kühlen und/oder Beheizen einer in die Analyse-Kassette eingefüllten Flüssigkeit, wobei das Zusatzmodul derart an den tragbaren Computer anschließbar ist, dass das Temperierelement aus der Energiequelle des Computers speisbar ist.

Zur Signal-Konditionierung ist z.B. ein Verstärker und/oder ein Analog-Digital-Wandler vorhanden.

Das Zusatzmodul ist vorzugsweise ohne eigene oder eingebaute Anzeigeeinrichtung ausgeführt. Zur Anzeige eines Messergebnisses kann die Anzeigeeinrichtung, z.B. der Monitor oder das Display, des tragbaren Computers genutzt werden.

Für Vorteile und vorzugsweise Ausgestaltungen des Zusatzmoduls, des tragbaren Computers, der digitalen Schnittstelle, der Analyse-Kassette und der Elektroden gelten die im Zusammenhang mit der Analyse-Kassette und der Vorrichtung nach der Erfindung oben gemachten Ausführungen analog.

Das Zusatzmodul ist vorzugsweise derart an, den tragbaren Computer anschließbar, dass die zum Aufprägen der Spannung und/oder des Stroms notwendige Energie der Energiequelle des Computers, insbesondere einer Batterie oder einem Akkumulator, entnehmbar ist.

Für eine kompakte Ausgestaltung kann das Zusatzmodul eine Ausnehmung zum Einschieben der Analyse-Kassette aufweisen.

Im Hinblick auf eine kompakte Ausführung ist es auch von Vorteil, dass die Schnittstelle eine PCMCIA-Schnittstelle ist.

Nach einer bevorzugten Weiterbildung kommt in dem Zusatzmodul das Temperierelement in großflächigen thermischen Kontakt mit der Analyse-Kassette. Mit dem Temperierelement, das vorzugsweise von dem tragbaren Computer zur Durchführung mehrerer Aufheiz- und Abkühlphasen ansteuerbar ist, kann in einer in der Analyse-Kassette befindlichen Flüssigkeit eine Polymerase-Ketten-Reaktion getriggert werden.

Das Temperierelement ist insbesondere ein Peltierelement.

Außerdem bevorzugt weist das Zusatzmodul ein elektrisch betreibbares Mittel zum Ausüben einer Kraft auf die Analyse-Kassette auf. Hinsichtlich Zweck, Vorteile und vorzugsweiser Ausgestaltungen dieses Mittels sei auf die weiter oben gemachten Ausführungen verwiesen.

Im Rahmen der Beschreibung liegt auch noch die Erkenntnis, dass die beschriebene Analyse-Kassette mit folgendem Verfahren in einfacher Weise herstellbar ist:
a) Ein Kunststoff-Formteil wird gefertigt, vorzugsweise durch Pressen;
b) in dem Kunststoff-Formteil werden mehrere Arbeitselektroden befestigt;
c) in dem Kunststoff-Formteil werden mehrere Gegenelektroden angebracht;
d) die Arbeitselektroden werden an dem Kunststoff-Formteil hängend in unterschiedliche, spezifische Biopolymere enthaltende Flüssigkeiten eingetaucht;
e) das Kunststoff-Formteil wird derart gefaltet, dass die Arbeitselektroden über zugeordneten Gegenelektroden zu liegen kommen und
f) auf einander zu liegen kommende Wandteile des Kunststoff-Formteils werden unter Bildung eines dichten Gehäuses der Analyse-Kassette miteinander verklebt oder verschweißt.

Dieses Verfahren ist auch zur Herstellung anderer Analyse-Kassetten als solcher für den elektrochemischen Nachweis einer in einer Flüssigkeit befindlichen Nukleotidsequenz verwendbar.

Bevorzugt wird bei dem Verfahren nach der Erfindung das Kunststoff-Formteil mit mehreren Vertiefungen gefertigt, wobei insbesondere für jede Gegenelektrode eine Vertiefung vorgesehen wird.

Mit dem Herstellungsverfahren sind Analyse-Kassetten in großer Stückzahl kostengünstig herstellbar, wobei die Schritte a) bis c) sowie e) und f) auch für Analyse-Kassetten, die für unterschiedliche Nachweiszwecke hergerichtet werden sollen, einheitlich durchführbar sind. Das heißt z.B., dass ein Oberteil und ein Unterteil des Kunststoff-Formteils kostengünstig in nur einer Variante in hoher Stückzahl herstellbar sind. Erst durch den nachträglichen Arbeitsschritt d) des Eintauchens oder Tränkens der Arbeitselektroden werden die Analyse-Kassetten ihrem späteren Verwendungszweck angepasst, so dass kurzfristig auf aktuelle Anforderungen reagiert werden kann oder auch kleinere Stückzahlen für Sonderanwendungen kostengünstig herstellbar sind.

Ausführungsbeispiele für die Vorrichtung nach der Erfindung, die Analyse-Kassette, das Zusatzmodul sowie für das Herstellungsverfahren werden nachfolgend anhand der Figuren 1 bis 7 näher erläutert. Es zeigen:
- FIG 1: eine schematische und perspektivische Gesamtansicht einer Vorrichtung nach der Erfindung,
- FIG 2: eine Analyse-Kassette der Vorrichtung nach Figur 1 im Detail (Längsschnitt),
- FIG 3: ein Zusatzmodul der Vorrichtung der Figur 1 im Detail (Längsschnitt),
- FIG 4: das Zusatzmodul der Figur 3 mit eingeschobener Analyse-Kassette der Figur 2,
- FIG 5: einen mit der Vorrichtung nach Figur 1 erfassbaren Signalverlauf,
- FIG 6: eine schematische Darstellung eines Satzes von Analyse-Kassetten sowie deren Anwendung in einer hierarchischen Vorgehensweise, und

- FIG 7 - 11: ein Beispiel für ein Herstellungsverfahren nach der Erfindung.

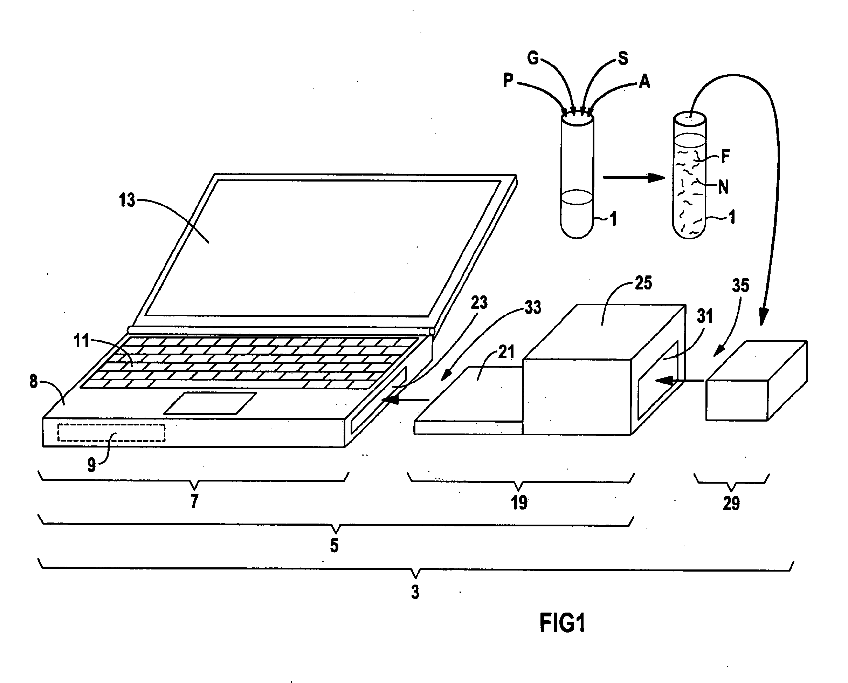

In **Figur 1** ist dargestellt, wie in ein Gefäß 1 eine Probe P, beispielsweise eine Blutprobe, zusammen mit einem Gerinnungshemmer G und gegebenenfalls einer die Zellwände aufbrechenden Substanz gegeben wird. Hinzugefügt wird außerdem ein spezielles Enzym S, das die nach Aufbrechen der Zellwände freigesetzten DNA-Stränge oder Nukleotidsequenzen an genau vordefinierten Stellen trennt ("enzymatische Schere"). Zugegeben werden außerdem Aminosäuren A, um nachfolgend eine Polymerase-Ketten-Reaktion durchzuführen und dadurch/eine Vervielfachung der in der Flüssigkeit F vorhandenen DNA-Abschnitte oder Nukleotidsequenzen N zu erreichen. Die DNA-Sequenzen können von Viren, Bakterien oder Krebszellen stammen. Außerdem können sie von "normalen" Zellen des menschlichen Körpers stammen, so dass auch Informationen aus dem Human-Genom gewinnbar sind, z.B. Hinweise auf eine Erbkrankheit oder auf eine Prädisposition für bestimmte Erkrankungen im Laufe des Lebens.

Zum elektrochemischen Nachweis der in der Flüssigkeit F befindlichen Nukleotidsequenzen N ist eine Vorrichtung 3 vorhanden, die eine tragbare Computereinrichtung 5 mit einem zusammenklappbaren mobilen Standard-Computer 7 aufweist. In einem Gehäuse 8 des Computers 7 ist als Energiequelle 9 eine Batterie oder ein Akkumulator vorhanden. Im Gehäuse 8 ist außerdem als Eingabemittel eine Tastatur 11 für Benutzereingaben sowie ein schwenkbares Display 13 zur Anzeige eines Nachweisergebnisses oder. einer Diagnose vorhanden. Der Akkumulator kann zeitweise mit einem (nicht gezeigten) Netz- oder Ladeteil in Verbindung stehen.

Die Vorrichtung 3 umfasst außerdem ein Zusatzmodul 19, das mit seinem flachen Steckteil 21 in eine Öffnung 23 im Gehäuse 8 des Computers 7 einsteckbar ist. Außerdem umfasst das Zusatzmodul 19 ein dickeres Aufnahmeteil 25 zur Aufnahme einer als Einmal-Kassette ausgebildeten Analyse-Kassette 29 ("Cartridge"), die ebenfalls Bestandteil der Vorrichtung 3 ist. Zum Andocken der Analyse-Kassette 29 an das Aufnahmeteil 25 weist letztgenanntes eine Ausnehmung 31 auf.

Die elektrische Verbindung des Zusatzmoduls 19 zum Computer 7 geschieht über eine als PCMCIA-Schnittstelle ausgebildete digitale Schnittstelle 33. Die Verbindung wird durch Einschieben des Steckteils 21 in die Öffnung 23 hergestellt, wobei sich entsprechende Steckkontakte schließen und wobei auch eine Verbindung des Zusatzmoduls 19 zur Energiequelle 9' realisiert wird.

Die elektrische Ankopplung der Analyse-Kassette 29 an das Zusatzmodul 19 geschieht über eine analoge Schnittstelle 35, deren Steckverbindung beim Andocken der Analyse-Kassette 29 in die Ausnehmung 31 hergestellt wird.

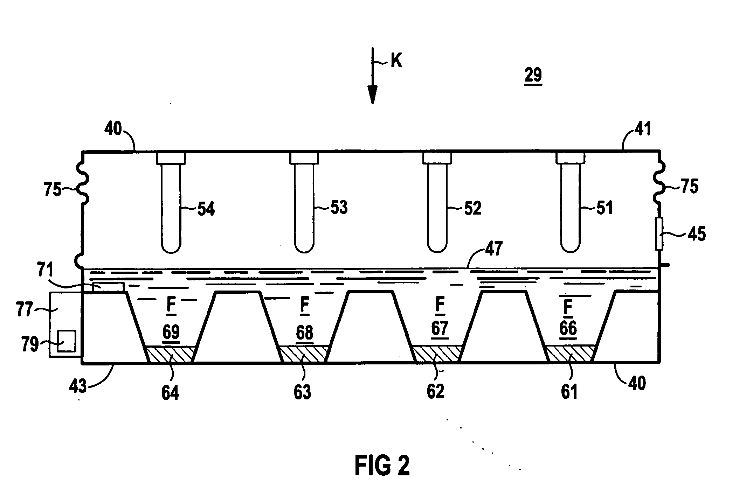

In **Figur 2** ist die etwa streichholzschachtelgroße Analyse-Kassette 29 im Detail dargestellt. Sie umfasst ein flüssigkeitsdichtes Gehäuse 40 mit einem deckelartigen Oberteil 41 und mit einem beckenartigen Unterteil 43. Das Gehäuse ist aus Kunststoff, beispielsweise nach dem weiter unten beschriebenen Herstellungsverfahren, hergestellt. Die Abmessungen der Analyse-Kassette 29 betragen etwa 50 mm x 35 mm x 12 mm (Dicke). Es sind auch Produktvarianten mit einer verkleinerten Kassette möglich.

In einem linken Wandteil des Oberteils 41 ist ein elastisches Wandelement 45 integriert, welches von einer Kanüle durchstechbar ist, um wenige µl der Flüssigkeit F in das Gehäuse 40 einzubringen. Das Wandelement 45 ist beispielsweise eine durchstechbare Membran, wie sie bei Infusionsflaschen Verwendung findet. Mit einer Injektionsspritze wurde die vorbereitete Flüssigkeit F in das Innere der Analyse-Kassette 29, also in die Analysekammer, bis zu einem Pegel 47 eingebracht.

Anstelle des elastischen Wandelements 45 können auch andere, beispielsweise ventilbasierte Zufuhrmittel, vorhanden sein, die einen Rückfluß der eingebrachten Flüssigkeit F aus der Analysekammer verhindern. Wichtig ist, dass die als Einmal-Kassette ausgebildete Analyse-Kassette 29 im Lieferzustand dicht versiegelt ist, wodurch sich eine gute Lagerfähigkeit auch bei höchsten hygienischen Anforderungen ergibt.

Im Inneren der Analyse-Kassette 29 befindet sich ein zur Durchführung des elektrochemischen Nachweisverfahrens notwendiger Elektrodensatz, umfassend vier am Oberteil 41 angebrachte Arbeitselektroden 51, 52, 53, 54, vier am Unterteil 43 angebrachte vergoldete Gegenelektroden 61, 62, 63, 64, sowie eine gemeinsame Referenzelektrode 71. Die flach ausgebildeten Gegenelektroden 61, 62, 63, 64 sind bodenseitig in durch die Formgebung des Unterteils 43 entstandenen Vertiefungen 66, 67, 68, bzw. 69 positioniert.

Die aus einem Kohlefasermaterial oder einem anderen an sich hierfür bekannten Werkstoff gefertigten, stabartigen Arbeitselektroden 51, 52, 53, 54 sind mit unterschiedlichen Biopolymeren beschichtet, die unterschiedliche spezifische Affinitäten zu nachzuweisenden Nukleotidsequenzen N in der Flüssigkeit F aufweisen. Die Biopolymere oder Rezeptormoleküle fungieren als passende "Gegenstücke'' zu den gesuchten Nukleotidsequenzen. Werden die entsprechend vorbereiteten Arbeitselektroden 51, 52, 53, 54 mit der Flüssigkeit F in Kontakt gebracht, dann lagern sich daran ausschließlich die exakt passenden Nukleotidsequenzen N an, so dass anschließend mittels einer elektrischen Auswertung die Anlagerung nachweisbar ist. Ein hierzu verwendbares Verfahren ist die sogenannte PSA ("potentiometric stripping analysis"). Hierzu wird zwischen je einer Arbeitselektrode 51, 52, 53, 54 und je einer zugehörigen Gegenelektrode 61, 62, 63 bzw. 64 ein Strom eingeprägt und dann zwischen der betreffenden Arbeitselektrode 51, 52, 53, 54 und der Referenzelektrode 71 eine allmählich ansteigende Spannung gemessen. Haben sich an der Arbeitselektrode 51, 52, 53, 54 Nukleotidsequenzen N der gesuchten Art angelagert, ist ein Verharren des Spannungsanstiegs bei 0,8 V zu beobachten, da bei dieser Spannung das im gesuchten DNA-Abschnitt enthaltene Guanin oxidiert. Die zeitliche Breite dieses Spannungsplateaus ist ein Maß für die Oxidierte Guaninmenge. Daraus kann der Rückschluss gezogen werden, ob in der untersuchten Probe DNA-Abschnitte der gesuchten Art in diagnostisch relevanter Menge vorhanden waren.

In das Oberteil 41 ist ein umlaufender Faltenbalg 75 integriert, so dass durch Ausüben einer externen Kraft K von oben auf das Oberteil 41 ein Absenken der Arbeitselektroden 51, 52, 53, 54 unter den Pegel 47 erreichbar ist.

An der rechten Seite des Gehäuses 40 ist ein elektrischer Kontaktblock 77 vorhanden, der auch als Halterung für ein als elektronischer Identifikations-Chip ausgebildetes Speichermodul 79 dient. Aus dem (für den Endanwender) nur lesbaren Speichermodul 79 sind von der Computereinrichtung 5 Daten betreffend die Art der Arbeitselektrode, eine unverwechselbare und nur einmal vergebene Seriennummer sowie zusätzliche Informationen betreffend den Hersteller, das Herstelldatum, das Haltbarkeitsdatum usw. sowie elektrodenspezifische Prozessparameter abrufbär.

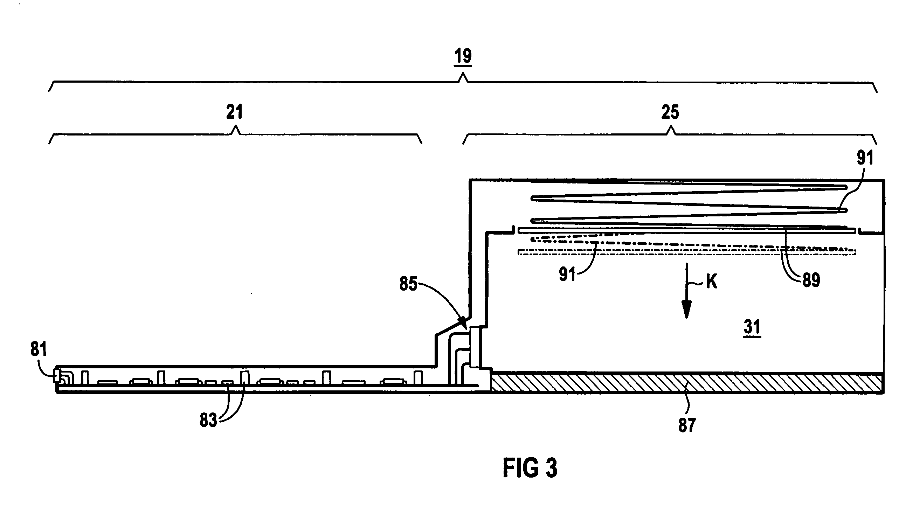

In **Figur 3** ist das mit einem leichten Kunststoff-Gehäuse gefertigte Zusatzmodul 19 nach der Erfindung genauer dargestellt. Zum Anschluß an die digitale Schnittstelle 33 des tragbaren Computers 7 weist das Zusatzmodul 19 endseitig an seinem Steckteil 21 eine Steckverbindung 81 auf. Im Innenraum des Steckteils 21 ist eine elektronische Schaltungsanordnung 83 vorhanden, die u.a. zur Durchführung der elektrochemischen Analyse benötigte spezifische Elektronikkomponenten, wie A/D-Umsetzer, D/A-Umsetzer, Stromquellen oder Spannungsquellen und Strom- bzw. Spannungsmesser umfassen. In dem aus dem Computer 7 herausragenden und damit für den Benutzer zugänglichen Aufnahmeteil 25 ist ein Peletierelement 87 bodenseitig integriert, welches die für die PCR erforderliche wiederholte kontrollierte Erwärmung und die anschließende Abkühlung ermöglicht. Elektronische Komponenten für die Ansteuerung des Peltierelements 87 sind im Steckteil 21 angebracht. Der Computer 7 sorgt für die Energieversorgung des Peletierelements 87 und übernimmt die Steuerung des zeitlichen Temperaturverlaufs.

In die Ausnehmung 31 des Zusatzmoduls 19 ist die Analyse-Kassette 29 der Figur 2 von links einschiebbar, bis diese mit ihrem Kontaktblock 77 in einen Steckverbinder 85 einrastet. Durch das Einrasten dient der Steckverbinder 85 außerdem als Haltevorrichtung für die Analyse-Kassette 29. Durch Herstellen der Steckverbindung werden die Elektroden 51, 52, 53, 54, 61, 62, 63, 64, 71 im Inneren der Analyse-Kassette 29 mit der elektronischen Schaltungsanordnung 83 im Zusatzmodul 19 verbunden.

Die zur Durchführung der elektrochemischen Analyse benötigten Elektronikkomponenten, insbesondere die zur Konditionierung und gegebenenfalls Auswertung vorhandenen Komponenten im Zusatzmodul 19, sind elektromagnetisch abgeschirmt, z.B. durch ein oder mehrere Bleche und/oder durch einen Faraday-Käfig.

Zur störungsfreien Trennung des Zusatzmoduls 19 bzw. dessen Elektronikkomponenten vom Computer 7 sind gesonderte und getrennte Masseführungen vorhanden. Mit gleichem Ziel können zur Signalübertragung Optokoppler vorhanden sein.

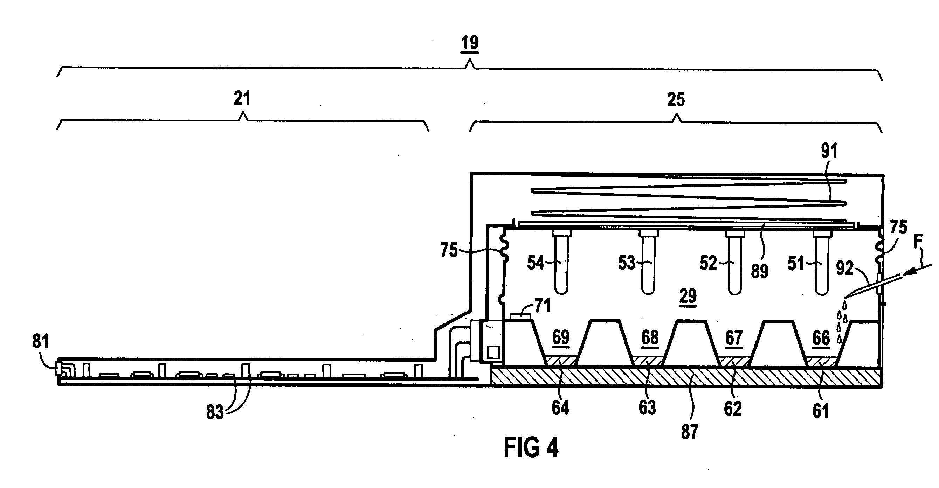

Das Zusatzmodul 19 zusammen mit der eingeschobenen Analyse-Kassette 29 ist in **Figur 4** gezeigt. Beim Einschieben der Analyse-Kassette 29 in die Ausnehmung 31 wurde eine an der Oberseite der Ausnehmung 31 im Aufnahmeteil 25 angeordnete Platte 89 aus ihrer in Figur 3 dargestellten gestrichelten Position entgegen der Kraft eines Formgedächtniselements 91 nach oben gedrückt. Das Formgedächtniselement 91 ist als mäanderförmiges Metallband aus SMA ("shape memory alloy" oder Metalllegierung mit Formgedächtnis, z.B. Nitinol) ausgeführt. Der elektrische Widerstand des Metallbands wird nach Abschluß der PCR benutzt, um das Metallband durch Anlegen einer elektrischen Spannung oder Einprägen eines elektrischen Stroms zu erwärmen. Hierdurch strebt das Formgedächtniselement 91 in eine ursprüngliche Form zurück, die ihm während der Herstellung durch eine spezielle Behandlung vorgegeben wurde. Bei diesem Zurückstreben in die ursprüngliche Form erzeugt die mit dem Formgedächtniselement 91 verbundene Platte 89 die externe Kraft K (siehe Figur 3), wodurch der Faltenbalg 75 gestaucht wird und wodurch die Arbeitselektroden 51, 52, 53, 54 in die Flüssigkeit F eintauchen, die zuvor mittels einer Kanüle 92 durch das elastische Wandelement 45 hindurch in die Analysekammer eingebracht wurde.

Der Betrieb der Vorrichtung 3 vollzieht sich also in folgenden Schritten:
1. Präparation einer Probe P zu einer Flüssigkeit F,
2. Andocken der gewünschten Analyse-Kassette 29 an die Computereinrichtung 5,
3. Durchführung der Polymerase-Ketten-Reaktion unter Verwendung des Peletierelements 87, soweit erforderlich,
4. Eintauchen der Arbeitselektroden 51, 52, 53, 54 in die Flüssigkeit F durch Aktivierung des Formgedächtniselements 91,
5. elektrochemischer Nachweis mittels PSA oder DPV ("differential pulsed voltametry").

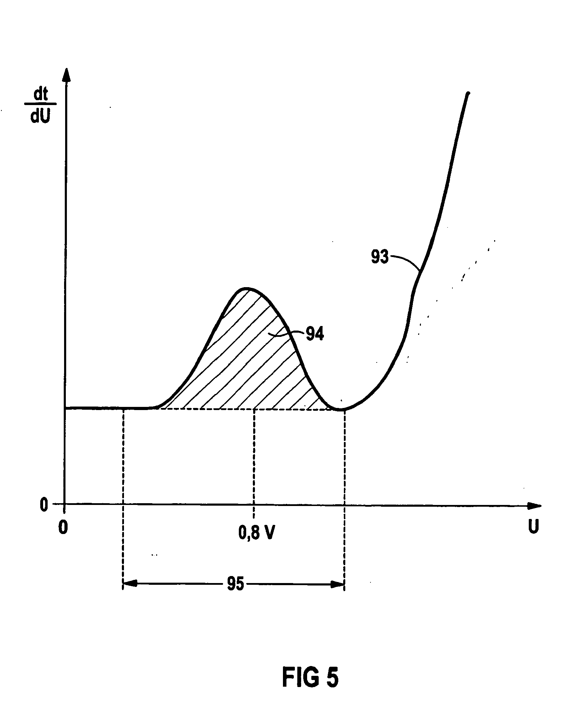

In **Figur 5** ist beispielhaft eine Kurve 93 dargesellt, wie sie sich im Rahmen der elektrochemischen Analyse ergibt. Im Beispiel wurde zwischen einer Arbeitselektrode und einer Gegenelektrode ein sehr kleiner Konstantstrom im Bereich zwischen 1 pA und 1 nA eingespeist und der zeitliche Spannungsverlauf U(t) zwischen der Referenzelektrode und der Arbeitselektrode aufgezeichnet. Anschließend wurde die Ableitung dt/dU gebildet, die zur Kurve 93 führte. Bei Anlagerung von DNA-Bruchstücken der gesuchten Art an der Arbeitselektrode zeigt sich durch die Oxidation von Guanin ein deutliches Maximum im Bereich von 0,8 V, wobei die Fläche 94 unter dem Maximum proportional zur Menge des Guanins ist. In dem in Figur 5 beispielhaft dargestellten Ergebnis einer PSA ist ein Messbereich 95 dargestellt, an den sich linksseitig ein Redox-Bereich und rechtsseitig ein Elektrolyse-Bereich anschließt.

Alternativ zur PSA als galvanostatischem Verfahren kann ein potentiostatisches Verfahren, vorzugsweise DPV, zum Einsatz kommen. Potentiostatische Verfahren sind deutlich robuster gegenüber Störeinflüssen und liefern somit im praktischen Einsatz das bessere Signal-/Noise-Verhältnis. Im Gegensatz zum mit Konstantstrom angeregten "Galvanostaten" arbeiten "Potentiostaten" mit Konstantspannung.

Beispielsweise wird der Messzelle zwischen der Referenzelektrode und der Gegenelektrode durch eine steuerbare niederohmige Spannungsquelle ein stufenweise ansteigendes Spannungspotential im Bereich von etwa 0,6 V bis 1,2 V aufgeprägt. Gemessen wird bei jeder Spannungsstufe und für eine definierte Zeit der über die Arbeitselektrode gegen Masse abfließende Strom. Der Kurvenverlauf des gewonnenen Signals entspricht dem der PSA (siehe Figur 5), wenn man auf der y-Achse dt/dI und auf der x-Achse den Strom I aufträgt.

Es können auch mehrere Arbeitselektroden mit einem der jeweiligen Elektrode zugeordneten Strommessgerät parallel in einem einzigen Messablauf gemessen werden. Anders ausgedrückt: Ordnet man jeder Elektrode ihr eigenes Amperemeter zu, so kann man in einem Messablauf alle Arbeitselektroden parallel auswerten. Grenzen werden hier nur durch den durch die Analyse-Kassette 29 fließenden Summenstrom und die Datenerfassungsgeschwindigkeit der verwendeten Elektronik gesetzt. Das bedeutet gegenüber den häufig im Laborbereich anzutreffenden Lösungen mit Multiplexern zur Umschaltung zwischen den Elektroden und sequentieller Abarbeitung aller Elektroden einen immensen Zeitvorteil, da ein Messzyklus immerhin etwa 45 sec. in Anspruch nimmt.

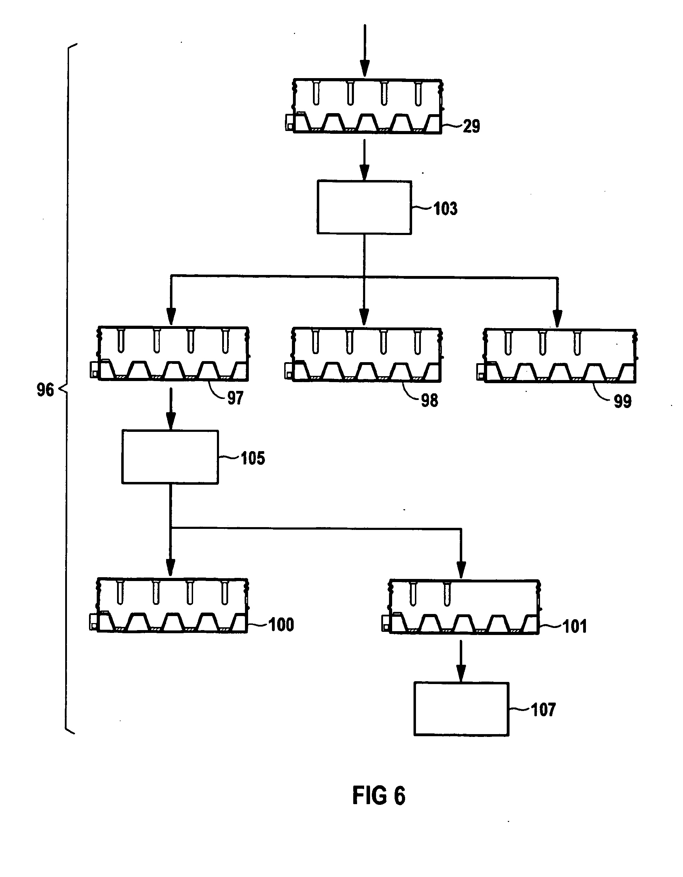

**Figur 6** zeigt schematisch einen Satz 96 mehrerer Analyse-Kassetten 29, 97, 98, 99, 100, 101, die untereinander baugleich sind, so dass sie alle in das Zusatzmodul 19 einführbar sind. Die Analyse-Kassetten 29, 97, 98, 99, 100, 101 unterscheiden sich dadurch voneinander, dass sie zum Nachweis voneinander unterschiedlicher Nukleotidsequenzen N bzw. zum Nachweis voneinander unterschiedlicher Kombinationen von Nukleotidsequenzen N geeignet sind. Im Beispiel beginnt ein detailliertes Nachweisverfahren durch Verwendung einer ersten Analyse-Kassette 29, die im Ergebnis auf dem Display 13 eine Grobdiagnose 103 zur Anzeige bringt. Auf dem Display 13 wird dem Nutzer außerdem in Abhängigkeit vom Ergebnis der Grobdiagnose 103 angezeigt, welche Analyse-Kassette,97 für eine gegebenenfalls gewünschte Feindiagnose 105 als nächstes in das Zusatzmodul 19 einzustecken ist. Die Feindiagnose 105 wird ebenfalls auf dem Display 13 angezeigt. Die hierarchische Anwendung der Analyse-Kassetten 29, 97, 98, 99, 100, 101 wird in Figur 6 auch noch für eine sich anschließende Feinstdiagnose 107 mittels der Analyse-Kassette 101 angedeutet.

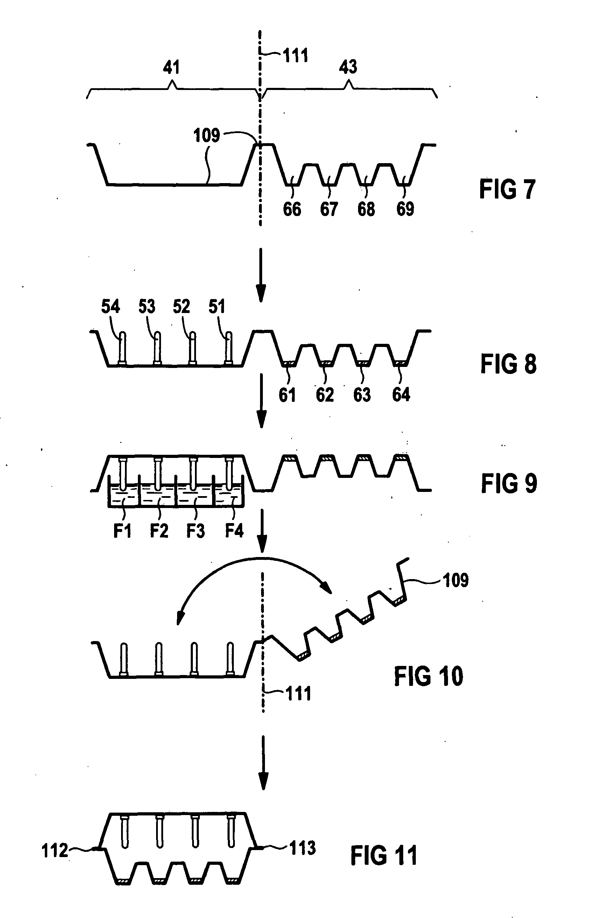

In den **Figuren 7 bis 11** ist ein kostengünstiges Herstellungsverfahren für die Analyse-Kassette 29 veranschaulicht. Das Verfahren beginnt damit (Figur 7), dass ein Kunststoff-Formteil 109 gepresst wird, in dem das Oberteil 41 und das Unterteil 43 des Gehäuses 40 einstückig zusammengefasst ist. Ziel des Herstellungsverfahrens ist es, die Analyse-Kassette 29 nach Art einer zusammenklappbaren Blisterverpackung herzustellen. Um das Zusammenklappen zu gewährleisten, ist das Kunststoff-Formteil 109 entlang einer Mittenebene 111 knickbar.

Vor dem Knicken werden aber zunächst in einem zweiten Schritt (**Figur 8**)die Arbeitselektroden 51, 52, 53, 54 sowie die zugehörigen Gegenelektroden 61, 62, 63, 64 angebracht. Anschließend werden im dritten Schritt (**Figur 9**)die Arbeitselektroden 51, 52, 53, 54 an dem Kunststoff-Formteil hängend in unterschiedliche, spezifische Biopolymere enthaltende Bäder mit Flüssigkeiten F1, F2, F3, F4 eingetaucht, bis eine ausreichende Beschichtung oder Benetzung erreicht ist.

Daran anschließend (**Figur 10**) wird das Kunststoff-Formteil 109 derart gefaltet, dass die Arbeitselektroden 51, 52, 53, 54 über zugeordneten Gegenelektroden 61, 62, 63, 64 zu liegen kommen. Nach dem Zusammenklappen werden das Oberteil 41 und das Unterteil 43 in einem letzten Schritt (**Figur 11**) an Rändern 112, 113 dicht verklebt oder mit Ultraschall verschweißt. Daraus ergibt sich eine gute Lagerfähigkeit auch unter ungünstigen Lager- und Klimabedingungen.

## Patentansprüche

1. Vorrichtung (3) zum elektrochemischen Nachweis einer in einer Flüssigkeit (F) befindlichen Nukleotidsequenz (N), mit einer Arbeitselektrode (51, 52, 53, 54), an die ein Biopolymer gebunden ist, welches eine spezifische Affinität zu der nachzuweisenden Nukleotidsequenz (N) aufweist, und mit einer Gegenelektrode (61, 62, 63, 64),
**gekennzeichnet durch**
a) eine austauschbare Analyse-Kassette (29) zum Einfüllen der Flüssigkeit (F), wobei die Arbeitselektrode (51, 52, 53, 54) und die Gegenelektrode (61, 62, 63, 64) in der Analyse-Kassette (29) angeordnet und mit der Flüssigkeit (F) in Kontakt bringbar sind,
b) eine tragbare, mit der Analyse-Kassette (29) elektrisch verbindbare Computereinrichtung (5) mit einem Display (13) und mit einer netzunabhängigen Energiequelle (9), wobei die Computereinrichtung (5) derart ausgebildet ist, dass den Elektroden (51, 52, 53, 54, 61, 62, 63, 64) eine Spannung und/oder ein Strom aufprägbar ist, dass ein von der Anlagerung der nachzuweisenden Nukleotidsequenz (N) auf der Arbeitselektrode (51, 52, 53, 54) herrührendes Strom- und/oder Spannungssignal erfassbar ist und dass ein daraus resultierendes Nachweisergebnis auf dem Display (13) darstellbar ist, und
c) ein aus der Energiequelle (9) gespeistes Temperierelement zum Kühlen und/oder Beheizen der Flüssigkeit (F) in der Analyse-Kassette (29).

2. Vorrichtung (3) nach Anspruch 1,
**dadurch gekennzeichnet, dass** die Computereinrichtung (5) derart ausgebildet ist, dass unter Verwendung ihrer Energiequelle (9) die Spannung bzw. der Strom aufprägbar ist.

3. Vorrichtung (3) nach Anspruch 1 oder 2,
**dadurch gekennzeichnet, dass** das Temperierelement ein Peltierelement (87) ist.

4. Vorrichtung (3) nach einem der Ansprüche 1 bis 3,
**dadurch gekennzeichnet, dass** die Computereinrichtung (5) einen Computer (7) mit einem Eingabemittel und dem Display (13) sowie ein an eine digitale Schnittstelle (33) des Computers (7) anschließbares Zusatzmodul (19) aufweist, das mit der Analyse-Kassette (29) über eine analoge Schnittstelle (35) verbindbar ist.

5. Vorrichtung (3) nach Anspruch 4,
**dadurch gekennzeichnet, dass** die digitale Schnittstelle (33) eine PCMCIA-Schnittstelle ist.

6. Vorrichtung (3) nach Anspruch 4 oder 5,
**dadurch gekennzeichnet, dass** der Computer (7) ein mobiler Standard-Computer, insbesondere ein Laptop, ein Notebook oder ein während der Bedienung zum Halten in der Hand vorgesehener Computer, z.B. ein Palmtop, ist.

7. Vorrichtung (3) nach einem der Ansprüche 4 bis 6,
**dadurch gekennzeichnet, dass** das Zusatzmodul (19) zumindest teilweise in ein Gehäuse (8) des Computers (7) einsteckbar ist.

8. Vorrichtung (3) nach einem der Ansprüche 4 bis 7,
**dadurch gekennzeichnet, dass** das Zusatzmodul (19) eine Ausnehmung (31) zum Einschieben der Analyse-Kassette (29) aufweist.

9. Vorrichtung (3) nach Anspruch 3 und nach einem der Ansprüche 4 bis 8,
**dadurch gekennzeichnet, dass**
das Temperierelement derart im Zusatzmodul (19) angebracht ist, dass es in großflächigen thermischen Kontakt mit der eingeschobenen Analyse-Kassette (29) kommt.

10. Vorrichtung (3) nach einem der Ansprüche 4 bis 9,
**gekennzeichnet durch**
ein im Zusatzmodul (19) angebrachtes, elektrisch betreibbares Mittel zum Ausüben einer Kraft (K) auf die Analyse-Kassette (29).

11. Vorrichtung (3) nach Anspruch 10,
**dadurch gekennzeichnet, dass**
das Mittel zum Ausüben einer Kraft (K) ein Element (91) mit einer Formgedächtnislegierung umfasst, wobei insbesondere das Element (91) bei einer Erwärmung die Rückkehr in einen herstellungsbedingten Ausgangszustand anstrebt und dabei die Kraft (K) erzeugt.

12. Vorrichtung (3) nach Anspruch 8 und 11,
**dadurch gekennzeichnet, dass**
das Mittel zum Ausüben einer Kraft (K) derart im Zusatzmodul (19) angeordnet ist, dass das Mittel bei Einschieben der Analyse-Kassette (29) in die Ausnehmung (31) im Zusatzmodul (19) gestaucht wird.

13. Vorrichtung (3) nach einem der Ansprüche 1 bis 12,
**dadurch gekennzeichnet, dass**
die Computereinrichtung (5) derart ausgebildet ist, dass unter Verwendung des erfassten Strom- und/oder Spannungssignals eine Diagnose hinsichtlich eines in der Flüssigkeit (F) befindlichen Krankheitserregers oder hinsichtlich einer in der Flüssigkeit (F) befindlichen krankhaften Körperzelle erstellbar und auf dem Display (13) anzeigbar ist.

## Claims

1. Apparatus (3) for the electrochemical detection of a nucleotide sequence (N) contained in a fluid (F), said apparatus (3) having a working electrode (51, 52, 53, 54) to which is bound a biopolymer that has a specific affinity with the nucleotide sequence (N) which is to be detected, and having a counter-electrode (61, 62, 63, 64),
**characterised by**
a) a replaceable analysis cassette (29) for introducing the fluid (F), with the working electrode (51, 52, 53, 54) and the counter-electrode (61, 62, 63, 64) being arranged in the analysis cassette (29) and able to be brought into contact with the fluid (F),
b) a portable computer entity (5) which is electrically connectable to the analysis cassette (29) and has a display (13) and an energy source (9) that is independent of a mains electricity supply,
wherein the computer entity (5) is embodied in such a way that a voltage and/or a current can be impressed on the electrodes (51, 52, 53, 54, 61, 62, 63, 64), that a current and/or a voltage signal generated as a result of the nucleotide sequence (N) that is to be detected being deposited on the working electrode (51, 52, 53, 54) can be recorded, and that a detection result produced therefrom can be presented on the display (13), and
c) a temperature-regulating element for cooling and/or heating the fluid (F) in the analysis cassette (29), said temperature-regulating element being fed from the energy source (9).

2. Apparatus (3) according to claim 1,
**characterised in that** the computer entity (5) is embodied in such a way that the voltage and/or current can be impressed using its energy source (9).

3. Apparatus (3) according to claim 1 or 2,
**characterised in that** the temperature-regulating element is a Peltier element (87).

4. Apparatus (3) according to one of claims 1 to 3,
**characterised in that**
the computer entity (5) has a computer (7) having an input means and the display (13) as well as an add-on module (19) which can be connected to a digital interface (33) of the computer (7) and can be connected to the analysis cassette (29) via an analogue interface (35).

5. Apparatus (3) according to claim 4,
**characterised in that**
the digital interface (33) is a PCMCIA interface.

6. Apparatus (3) according to claim 4 or 5,
**characterised in that**
the computer (7) is a mobile standard computer, in particular a laptop, a notebook or a computer that is designed to be held in the hand during operation, e.g. a palmtop.

7. Apparatus (3) according to one of claims 4 to 6,
**characterised in that**
the add-on module (19) can be inserted at least partially into a housing (8) of the computer (7).

8. Apparatus (3) according to one of claims 4 to 7,
**characterised in that**
the add-on module (19) has a recess (31) allowing the analysis cassette (29) to be inserted.

9. Apparatus (3) according to claim 3 and according to one of claims 4 to 8,
**characterised in that**
the temperature-regulating element is installed in the add-on module (19) in such a way that it is brought into thermal contact with the inserted analysis cassette (29) over a large surface area.

10. Apparatus (3) according to one of claims 4 to 9,
**characterised by**
an electrically operable means installed in the add-on module (19) for the purpose of exerting a force (K) on the analysis cassette (29).

11. Apparatus (3) according to claim 10,
**characterised in that**
the means for exerting a force (K) includes an element (91) comprising a shape memory alloy, wherein the element (91) attempts, in particular upon being heated, to return to a manufacture-induced original state, generating the force (K) in the process.

12. Apparatus (3) according to claim 8 and 11,
**characterised in that**
the means for exerting a force (K) is arranged in the add-on module (19) in such a way that the means is compressed when the analysis cassette (29) is inserted into the recess (31) in the add-on module (19).

13. Apparatus (3) according to one of claims 1 to 12,
**characterised in that**
the computer entity (5) is embodied in such a way that a diagnosis in respect of a pathogen contained in the fluid (F) or in respect of a diseased body cell contained in the fluid (F) can be produced using the recorded current and/or voltage signal and displayed on the display (13).

## Revendications

1. Dispositif (3) pour le décèlement électrochimique d'une séquence nucléotidique (N) se trouvant dans un liquide (F), comprenant une électrode de travail (51, 52, 53, 54) à laquelle est relié un biopolymère qui présente une affinité spécifique par rapport à la séquence nucléotidique (N) à déceler, et comprenant une contre-électrode (61, 62, 63, 64),
**caractérisé par**
a) une cassette d'analyse échangeable (29) pour y remplir le liquide (F), l'électrode de travail (51, 52, 53, 54) et la contre-électrode (61, 62, 63, 64) étant disposées dans la cassette d'analyse (29) et pouvant être amenées en contact avec le liquide (F) ;
b) un dispositif informatique portable (5) qui peut être relié électriquement à la cassette d'analyse (29), comprenant un écran (13) et une source d'énergie (9) indépendante du réseau, le dispositif informatique (5) étant conçu de telle sorte qu'une tension et/ou un courant peut venir s'appliquer sur les électrodes (51, 52, 53, 54, 61, 62, 63, 64), de telle sorte qu'un signal de courant et/ou de tension provenant du dépôt de la séquence nucléotidique (N) à déceler sur l'électrode de travail (51, 52, 53, 54) et de telle sorte qu'un résultat du décèlement obtenu peut être représenté sur l'écran (13), et
c) un élément de traitement thermique alimenté par la source d'énergie (9) pour le refroidissement et/ou le réchauffement du liquide (F) dans la cassette d'analyse (29).

2. Dispositif (3) selon la revendication 1,
**caractérisé en ce que** le dispositif informatique (5) est conçu de telle sorte qu'en utilisant sa source d'énergie (9) la tension respectivement le courant peut venir s'appliquer.

3. Dispositif (3) selon la revendication 1 ou 2,
**caractérisé en ce que** l'élément de traitement thermique est un élément Peltier (87).

4. Dispositif (3) selon l'une quelconque des revendications 1 à 3,
**caractérisé en ce que** le dispositif informatique (5) présente un ordinateur (7) comprenant un moyen d'entrée et un écran (13), ainsi qu'un module supplémentaire (19) qui peut se raccorder à une interface numérique (33) de l'ordinateur (7), le module étant à même de se relier à la cassette d'analyse (29) via une interface analogique (35).

5. Dispositif (3) selon la revendication 4,
**caractérisé en ce que** l'interface numérique (33) est une interface PCMCIA.

6. Dispositif (3) selon la revendication 4 ou 5,
**caractérisé en ce que** l'ordinateur (7) est un ordinateur standard mobile, en particulier un ordinateur portatif, un ordinateur bloc-notes ou bien un ordinateur prévu pour être tenu en main lors de son utilisation, par exemple un ordinateur de poche.

7. Dispositif (3) selon l'une quelconque des revendications 4 à 6,
**caractérisé en ce que** le module supplémentaire (19) peut venir s'enficher au moins en partie dans un logement (8) de l'ordinateur (7).

8. Dispositif (3) selon l'une quelconque des revendications 4 à 7,
**caractérisé en ce que** le module supplémentaire (19) présente un évidement (31) pour que la cassette d'analyse (29) vienne s'y insérer par glissement.

9. Dispositif (3) selon la revendication 3 et selon l'une quelconque des revendications 4 à 8,
**caractérisé en ce que** l'élément de traitement thermique vient s'appliquer dans le module supplémentaire (19) de telle sorte qu'il entre en contact thermique sur toute la surface avec la cassette d'analyse insérée par glissement (29).

10. Dispositif (3) selon l'une quelconque des revendications 4 à 9,
**caractérisé par** un moyen apte à un entraînement électrique appliqué dans le module supplémentaire (19) pour exercer une force (K) sur la cassette d'analyse (29).

11. Dispositif (3) selon la revendication 10,
**caractérisé en ce que** le moyen pour exercer une force (K) comprend un élément (91) possédant un alliage à mémoire de forme, dans lequel en particulier l'élément (91) tente de revenir, lors d'un réchauffement, à un état de départ imposé par la fabrication, et génère ainsi la force (K).

12. Dispositif (3) selon les revendications 8 et 11,
**caractérisé en ce que** le moyen pour exercer une force (K) est disposé dans le module supplémentaire (19) de telle sorte que le moyen est comprimé dans le module supplémentaire (19) lors de l'insertion par glissement de la cassette d'analyse (29) dans l'évidement (31).

13. Dispositif (3) selon l'une quelconque des revendications 1 à 12,
**caractérisé en ce que** le dispositif informatique (5) est conçu de telle sorte qu'en utilisant le signal enregistré de courant et/ou de tension, un diagnostic concernant un agent pathogène se trouvant dans le liquide (F) ou concernant une cellule somatique pathologique se trouvant dans le liquide (F) peut être établi et peut être affiché sur l'écran (13).
